Europäisches Patentamt

European Patent Office

Office européen des brevets

(19)

(11) Publication number: **0 478 333 A1**

# (12) EUROPEAN PATENT APPLICATION

(21) Application number: **91308810.0**

(22) Date of filing: **26.09.91**

(51) Int. Cl.⁵: **C12N 15/86,** C12N 15/79, C12N 15/67, C12N 15/47

(30) Priority: **28.09.90 US 590629**

(43) Date of publication of application:
**01.04.92 Bulletin 92/14**

(84) Designated Contracting States:
**AT BE CH DE DK ES FR GB GR IT LI NL SE**

(71) Applicant: **ELI LILLY AND COMPANY**
**Lilly Corporate Center**
**Indianapolis Indiana 46285 (US)**

(72) Inventor: **Grinnell, Brian William**
**3625 East 71st Street**
**Indianapolis, Indiana 46220 (US)**

(74) Representative: **Hudson, Christopher Mark et al**
**Erl Wood Manor**
**Windlesham Surrey GU20 6PH (GB)**

(54) **Gene selective expression with vectors containing protector and suppressor viral sequences.**

(57)    The present invention provides DNA compounds, recombinant DNA vectors, and methods for achieving selective expression of polpeptide products of interest in eukaryotic cells. The invention further provides DNA compounds which function as eukaryotic promoters as well as prokaryotic translational activation sequences.

EP 0 478 333 A1

Jouve, 18, rue Saint-Denis, 75001 PARIS

The high level expression of genes in eukaryotic cells is at present limited by the availability of eukaryotic expression systems which efficiently utilize the host cell transcriptional and translational machinery.

Impetus for the derivation of mammalian expression systems stems from the findings that many eukaryotic gene products, when expressed in bacterial systems, lack the appropriate post translational modifications required for functional properties. These modifications include glycosylation pattern, modification of side groups such as $\gamma$-carboxylation, precise proteolytic processing of precursors and proper formation of disulfide bridges. In addition, the folding (3 dimensional state) of proteins expressed in prokaryotes may be atypical of the translation product of eukaryotic cells. The result is often the production of molecules of less than optimal biological activity or no biological activity. Additionally, rigorous purification and refolding procedures are often required for recombinant DNA products which are expressed in prokaryotic cells.

The present invention solves many problems in the art by providing a method of eukaryotic expression vector construction in which genes encoding a useful substance are joined in frame to a VSV cDNA sequence which drives transcription of the gene or genes encoding said useful substance. An important aspect of the invention is the association of the promoter/gene transcription unit with a viral sequence, which prevents suppression of non-viral protein synthesis when the host cell is exposed to a virus or viral regulatory molecules which suppress expression of genes that are not protected by a protector sequence. The vectors of the invention have also been constructed to include enhancer sequences to further increase expression of the desired substances in eukaryotic cells.

A further aspect of the present invention is the use of VSV leader sequence cDNA to drive expression of products of interest in both prokaryotic and eukaryotic host cells.

The accompanying figures are briefly described below:

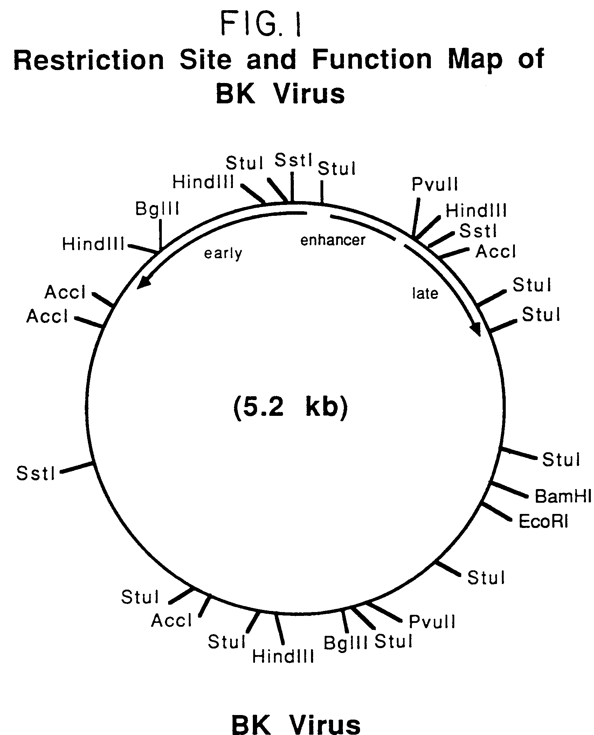

Figure 1 is a restriction site and function map of BK virus.

Figure 2 is a restriction site and function map of plasmid pBKE1.

Figure 3 is a restriction site and function map of plasmid pBKneol.

Figure 4 is a restriction site and function map of plasmid pSV2cat.

Figure 5 is a restriction site and function map of plasmid pLPcat.

Figure 6 is a restriction site and function map of plasmid pBLcat.

Figure 7 is a restriction site and function map of pUCLN$\beta$-1.

Figure 8 is a restriction site and function map of pUCLN$\beta$-2.

Figure 9 is a restriction site and function map of pUCLN$\beta$-5.

Figure 10 is a restriction site and function map of pUCLN$\beta$-4.

The present invention provides a method for the selective expression of products of interest in eukaryotic cells comprising culturing an eukaryotic host cell transformed with at least one recombinant DNA expression vector comprising a viral protector sequence positioned on said recombinant DNA vector such that the protector sequence is transcribed on the mRNA encoding said product of interest and a viral suppressor sequence positioned in proper reading frame with a second promoter such that the expression of the mRNA encoding said product of interest and comprising a viral protector sequence coincides with the expression of the viral suppressor.

A viral protector sequence is any DNA sequence of viral origin or the chemically synthesized equivalent thereof which when transcribed as part of a mRNA transcript renders that mRNA immune to viral suppression of non-viral protein synthesis. Selective expression of products of interest in eukaryotic host cells is attained when mRNA comprising a coding sequence for a product of interest and a viral protector sequence is present in an eukaryotic host cell which also contains viral suppressor. A viral suppressor sequence is any DNA or RNA sequence of viral origin or the chemically synthesized equivalent thereof which when expressed in an eukaryotic cell results in suppression of non-viral protein synthesis alone, or in conjunction with host cell factors.

The present invention teaches the isolation of protector sequences and suppressor sequences from viruses which suppress non-viral protein synthesis. The present invention further teaches the use of the protector and suppressor sequences in the construction of recombinant DNA vectors, which selectively express products of interest in eukaryotic host cells.

The protector sequence utilized to illustrate the present invention was isolated from Vesicular Stomatitis Virus (VSV). VSV is a negative strand RNA virus belonging to the Rhabdo virus group. The protector sequence isolated from VSV was obtained by preparing cDNA corresponding to the portion of the VSV genome which codes for the VSV leader sequence and part of the N gene.

Several plasmids were constructed to illustrate the promoter and protector functions of the VSV derived sequence. The illustrative plasmids disclosed herein to promote an understanding of the present invention are members of the pUCLN$\beta$ series. The pUC signifies that the E. coli replicon and ampicillin gene of this series of plasmids were derived from plasmid pUC13. The LN denotes the presence of the VSV leader (L) and a part of the N gene (N) coding region. The $\beta$ represents a truncated (codons 1-9 are deleted) $\beta$-galactosidase coding

region. Construction of the pUCLNβ series of plasmids as well as details of the various regulatory systems represented by this series of plasmids are described below.

The preferred source of the VSV protector/ promoter sequence is plasmid pUCLNβ-1 which has been deposited in the Northern Regional Research Center (NRRL), Agricultural Research Service, Peoria, Illinois 61604, and is publicly available under the accession number NRRL B-18715, deposited September 21, 1990. The sense (coding) strand of the cDNA prepared from VSV-derived sequence is set forth below.

<div align="center">LEADER</div>

```
ACGAAGACAAACAAACCATTATTATCATTAAAAGGCTCAGGAGAAACTTTAACA
                                                    ↑
                                                 CAP SITE
```

<div align="center">N GENE</div>

```
GTAATCAAAATGTCTGTTACAGTCAAGAGAATCATTGACAACACAGTCATAGTT

|_____|
     RIBOSOME BINDING SITE

CCAAAACTTCCTGCAAATGAGGATCC
           'BamHI'
```

The sequence depicted above as well as other viral protector or suppressor sequences can be synthesized from single stranded deoxyoligonucleotides by procedures well known in the art. The single stranded deoxyoligonucleotides can be synthesized with commercially available instruments, such as the 380A DNA Synthesizer marketed by Applied Biosystems (850 Lincoln Centre Drive, Foster City, CA 94404), which utilizes phosphoramidite chemistry. Other procedures for synthesizing DNA are also known in the art. The conventional modified phosphotriester method of synthesizing single stranded DNA is described in Itakura et al., 1977, Science 198:1056 and in Crea et al., 1978, Proc. Nat. Acad. Sci. USA 75:5765. In addition, a preferred method of synthesizing DNA is disclosed in Hsiung et al., 1983, Nucleic Acid Research 11: 3227 and Narang et al., 1980, Methods in Enzymology 68:90.

Skilled artisans realize that the promoter and protector functions of the above VSV-derived sequence are separable. The small size of the VSV-derived sequence does not impose significant limitations on expression vector construction. However, separation of promoter and protector functions is well within the skill of the art through either restriction enzyme digestion or alternatively, by chemical synthesis of portions of the VSV sequence disclosed above.

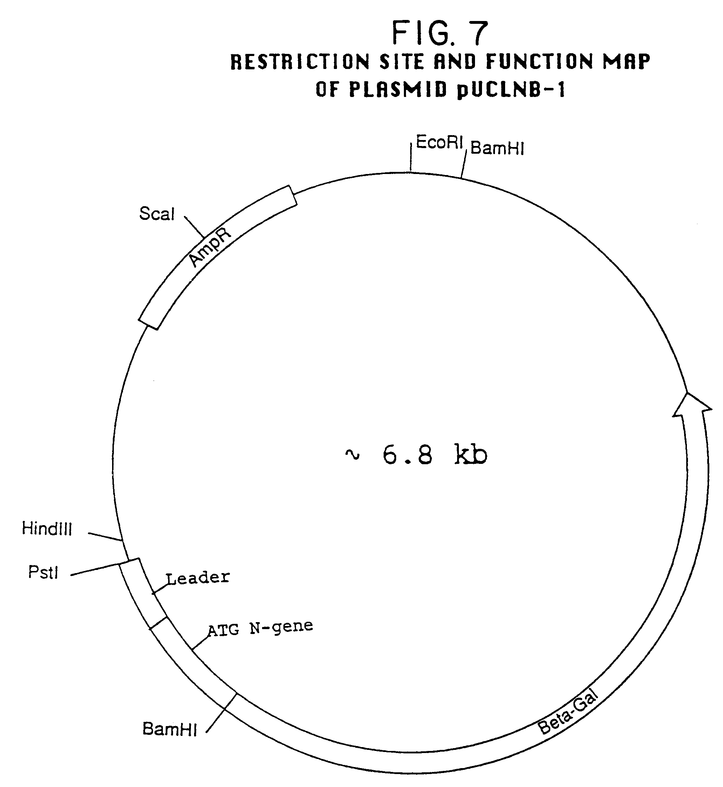

A restriction site and function map of plasmid pUCLNβ-1 is provided in Figure 7. Plasmid pUCLNβ-1 contains the leader sequence and a portion of the N gene fused in proper reading frame to the 10th codon of the β-galactosidase gene to allow transcription and translation of a truncated N gene/β-galactosidase fusion protein. The β-galactosidase gene product is enzymatically active despite its fusion to the truncated N gene. Thus the β-galactosidase gene provides a facile means of determining transcription and/or translation from regulatory sequences. β-galactosidase (β-gal) and chloramphenicol acetyl transferase (CAT) are commonly used reporter genes. Reporter genes are routinely used to monitor a promoter's ability to drive gene expression. The ability to monitor expression of a product of interest is especially useful in identifying further protector and suppressor sequences. The ability to monitor reporter gene expression driven by any given promoter allows the detection of protector sequences simply by monitoring the continuance of reporter gene expression following viral infection and the subsequent suppression of "non-protected", non-viral protein synthesis. Thus, protector sequences other than the VSV protector sequence are readily identified using vectors analogous to pUCLNβ-1.

Plasmid pUCLNβ-1 was constructed by ligating the BamHI restriction site in the VSV leader/N gene sequence to a BamH1 digested β-gal reporter gene and inserting the leader/N gene/β-gal into a plasmid pUC13 backbone. The use of β-galactosidase gene fusions for analyzing gene expression is well-known in the art. See Casadaban et al., 1983, "Methods in Enzymology", Vol. 100, pgs. 293-308. Plasmid pUCLNβ-1 also contains the E. coli replicon and an antibiotic resistance gene derived from plasmid pUC13. The addition of E. coli replication and selection functions to the plasmids of the present invention adds to the general utility of the invention by allowing replication and selection of these vectors in prokaryotic hosts. Plasmid pUCLNβ-1 also contains the SV40 poly A and splice sites located 3' to the β-gal coding region.

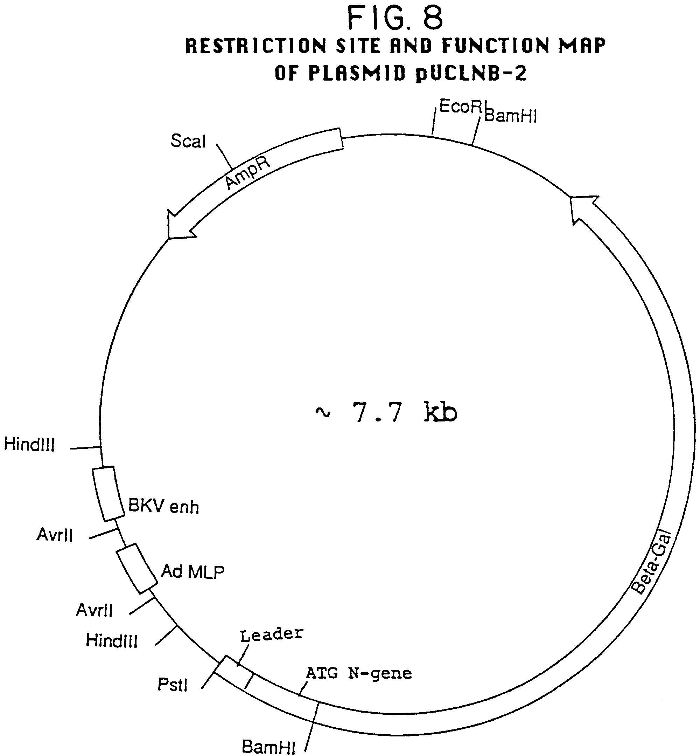

Plasmids pUCLNβ-2 and pUCLNβ-3 were constructed by insertion of an ~0.87 kb HindIII fragment comprising the BK enhancer and Adenovirus type II major late promoter into the HindIII site 5' to the VSV sequence in plasmid pUCLNβ-1. A restriction site and function map of plasmid pUCLNβ-2 is provided in Figure 8. Plasmid pUCLNβ-2 differs from pUCLNβ-3 only in the orientation of the ~0.87 kb HindIII fragment. In plasmid pUCLNβ-2 the insertion of the ~0.87 kb HindIII fragment of plasmid pBL-CAT into the HindIII site of plasmid pUCLNβ-1 results in the BK enhancer and Adenovirus type II late promoter being positioned to drive high level expression of the truncated N gene/β-galactosidase fusion protein. Transcription of the N gene/β-galactosidase message is driven in plasmid pUCLNβ-3 by the BK early promoter/enhancer. Thus, pUCLNβ-2 and pUCLNβ-3, while differing in the promoter utilized, are both eukaryotic expression vectors for the gene fusion product of the truncated N gene/β-galactosidase genes.

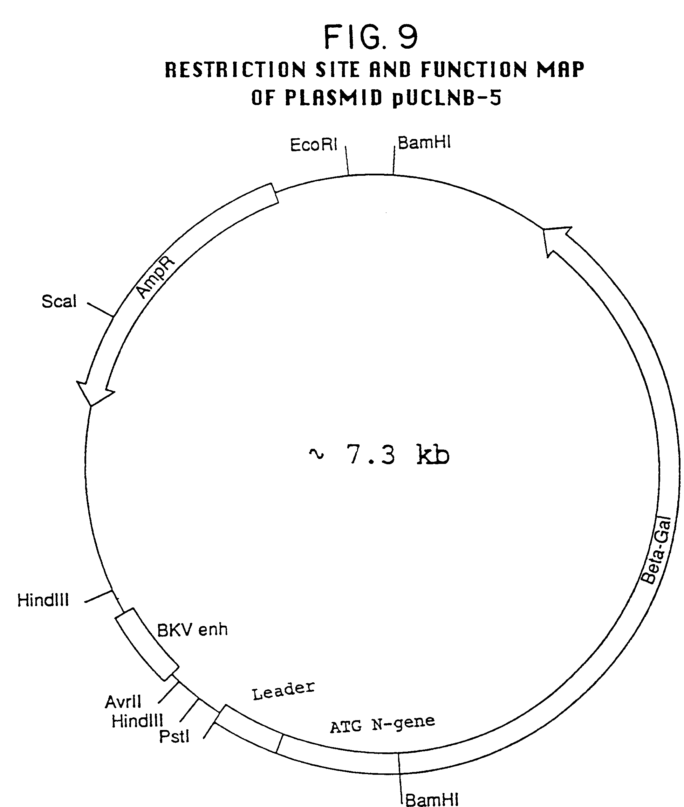

Plasmid pUCLNβ-5 was prepared by excising the ~373 bp AvrII fragment of plasmid pUCLNβ-2. Thus, plasmid pUCLNβ-5 does not contain the Adenovirus type II late promoter and the BK early promoter is improperly oriented to drive expression of the N gene/β-galactosidase fusion product. Expression of the N gene/β-galactosidase fusion product is driven by the VSV derived leader sequence. A restriction site and function map of plasmid pUCLNβ-5 is provided in Figure 9.

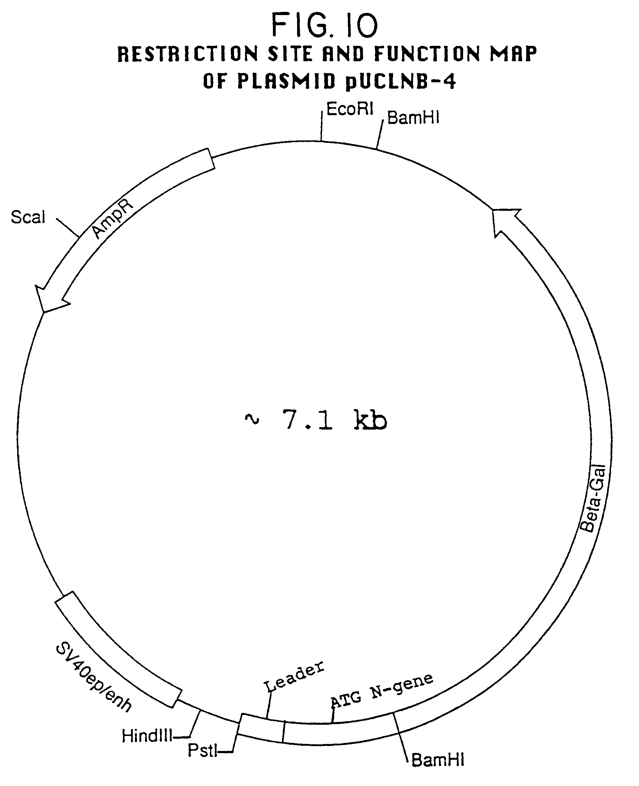

Plasmid pUCLNβ-4 was constructed by inserting the SV40 early promoter from plasmid pSV2-CAT (ATCC Number 37155) into ScaI/HindIII digested plasmid pUCLNβ₁. The SV40 early promoter was harvested from plasmid pSV2-CAT as an ~2122 bp ScaI/HindIII restriction fragment. The ~2122 bp ScaI/HindIII fragment was then ligated into the ~5021 bp ScaI/HindIII digestion fragment of plasmid pUCLNβ-1. Thus, plasmid pUCLNβ-4 is also capable of driving expression of the N gene/β-galactosidase fusion protein from the SV40 early promoter located 5' to the leader/N gene/ β-galactosidase. A restriction site and function map of plasmid pUCLNβ-4 is provided in Figure 10.

The relative levels of β-galactosidase produced by the plasmids pUCLNβ-1 and pUCLNβ-5 were determined in the 293 cell-line. For comparative purposes, the level of β-gal produced from the pUCLNβ plasmids was compared to β-gal production by pMC1924, a plasmid in which the strong SV40 early promoter/ enhancer drives expression of β-gal. The β-galactosidase assay was performed in substantial accordance with the method of Casadaban et al., 1983, Methods in Enzymology, Vol. 100, pgs. 293-308, the teachings of which are herein incorporated by reference.

Table 1

Expression of β-galactosidase from the VSV leader/N gene containing plasmids in human 293-cells

| Plasmid | Regulatory Region | Relative Levels of β-galactosidase |
|---------|-------------------|-----------------------------------|
| pMC1924 | SV40 early promoter/enhancer | 1 |
| pUCLNβ-1 | VSV leader and N gene | 0.19 |
| pUCLNB-5 | BK enhancer, VSV leader and N gene | 0.87 |

The data of Table 1 illustrates two important aspects of the present invention. First, the leader sequence of VSV functions as a polymerase II promoter. Secondly, the DNA polymerase II activity of the VSV leader sequence can be stimulated by the presence of an enhancer element. The ability of the VSV leader sequence to function as a polymerase II promoter is unexpected. VSV, a negative strand RNA virus, would not be expected to need or possess a DNA dependent RNA polymerase as nothing in the physiology of the virus directly requires RNA synthesis from a DNA template. Thus, the cDNA prepared from the VSV leader sequence provides a new and useful promoter for purposes of constructing eukaryotic expression vectors.

The ability to stimulate gene expression from the VSV-derived promoter by proper positioning of enhancer sequences on the expression vectors adds to the general utility of the present invention. The BK enhancer was used to illustrate the desirability of including enhancer sequences in the eukaryotic expression vectors of the present invention. Skilled artisans realize that enhancers other than the BK enhancer are also useful for purposes of the present invention. The utility of this aspect of the present invention is illustrated by the data of Table II.

Table II

Expression of β-galactosidase from VSV leader/N gene
containing plasmids in COS-1 cells

| Plasmid | Regulatory Region | Relative Levels of β-galactosidase or CAT |
|---|---|---|
| pMC1924 | SV40 early promoter and enhancer | 1 |
| pSV2-CAT | SV40 early promoter and enhancer | 1 |
| pBK-CAT | BK enhancer | 0.40 |
| pUCLNβ-1 | VSV leader and N gene | not detectable |
| pUCLNβ-5 | VSV leader and N gene and BK enhancer | 15.6 |

The data of Table II further illustrate the utility of the VSV leader sequence and N gene as a polymerase II promoter. Plasmids pMC1924 and pSV2-CAT were included as controls to establish the levels of β-galactosidase and chloramphenicol acetyl transferase respectively, which are driven by the SV40 early promoter and enhancer in human COS-1 cells. The high level of β-galactosidase expression by plasmid pUCLNβ-5 illustrates the promoter strength of the VSV-derived sequence in COS-1 cells and further illustrates the stimulatory effect which enhancer sequences exert on expression driven by the VSV-derived sequence.

The chloramphenicol acetyl transferase (CAT) assay was performed in substantial accordance with the teachings of Sambrook, Fritsch, and Maniatis, "Molecular Cloning - A Laboratory Manual, 2nd Ed", 1989, Cold Spring Harbor Laboratory Press, pages 16.59-16.62 (hereinafter Maniatis).

Plasmid pMC1924 (Nielsen, et al., 1983, P.N.A.S., Vol. 50, pgs. 5198-5202), which served as a control in Tables I and II, contains the rabbit β globin gene fused in frame to the 10th codon of the β-galactosidase gene driven by the SV40 early promoter.

The preceding section described the construction of illustrative expression vectors comprising the VSV leader sequence and N gene in the context of various other viral regulatory sequences. The following data and discussion illustrate the use of VSV-derived protector and suppressor sequences to achieve selective expression of products of interest in eukaryotic host cells.

Separate cultures of cell-line 293 were transfected with plasmid pUCLNβ-4 and pMC1924. Both plasmids contain the strong SV40 early promoter/enhancer driving expression of β-gal. Plasmid pUCLNβ-4 also contains the VSV protector sequence. The levels of β-gal produced from plasmids pMC1924 and pUCLNβ-4 were similar in the absence of suppressor activity. Three days following transfection the cultures were infected with VSV. β-gal activity and $^{14}$C-amino acid incorporation into protein were subsequently monitored to quantitate fusion protein activity and total protein synthesis respectively. Results of these studies are summarized in Table III.

## Table III

Effect of VSV Infection on the Level of β-galactosidase
Synthesis from Plasmids Containing the VSV
Leader and N Gene in 293 Cells

| Plasmid & β-galactosidase/ Total Protein | Time After Infection | % of Control β-galactosidase | Total Protein |
|---|---|---|---|
| pMC1924 & 1 | 0 | 100 | 100 |
| pUCLNβ-4 & 1 | 0 | 100 | 100 |
| pMC1924 & 1.18 | 2 | 94 | 80 |
| pUCLNβ-4 & 1.9 | 2 | 132 | 69 |
| pMC1924 & 1.29 | 5 | 45 | 35 |
| pUCLNβ-4 & 3.0 | 5 | 98 | 33 |

The data of Table III demonstrate the protection afforded mRNAs comprising the VSV-derived protector sequence from viral suppression of non-viral protein synthesis. The β-galactosidase activity in 293 cells containing pUCLNβ-4 remained at 98% of control levels at 5 hours post VSV infection, while β-galactosidase levels in 293 cells containing pMC1924 were at 45% of control at 5 hours post-VSV infection. Note also that the 98% of control and 45% of control levels of β-gal produced by 293 cells containing pUCLNβ-4 and pMC1924 respectively reflect a time point (5 hours post-VSV infection) when total cell protein synthesis had been reduced to about 35% of control levels by VSV suppression of non-viral protein synthesis.

The present invention provides a method for the selective expression of products of interest in eukaryotic host cells when a protector sequence is transcribed at a position 5′ to the sequence encoding the product of interest. Infection of cells containing the VSV-derived protector sequence with VSV was used merely to illustrate the invention. Skilled artisans realize that viral gene products alone, or in conjunction with host cell factors are responsible for suppression of non-viral protein synthesis. Thus, it is preferable to utilize a viral gene product to suppress non-viral protein synthesis without introduction of a virus into the host cell. Those skilled in the art of molecular biology also realize that only the viral gene encoding the gene product which suppresses non-viral protein synthesis is required to be present in the host cell for the protector sequence to function in the selective expression of mRNA sequences encoding useful substances. Viral gene products which suppress synthesis of non-viral proteins are referred to as suppressors. The isolation of genes encoding suppressors and the substitution of suppressor expression vectors for intact virus is a preferred embodiment of the present invention. The identification and utilization of further protector sequences and the corresponding suppressors is a further embodiment of the present invention.

The method of the present invention is not limited to VSV-derived protectors or suppressors. Isolation of the VSV protector sequence required the isolation of the VSV genome and reverse transcription of the negative strand to generate cDNA. The isolation of viral RNA and preparation of cDNA therefrom are techniques well-known in the art. See Sambrook, Fritsch, and Maniatis, "Molecular Cloning - A Laboratory Manual, 2nd Ed", 1989, Cold Spring Harbor Laboratory Press (hereinafter Maniatis).

Cloning cDNA libraries and the screening of the libraries for protector and suppressor sequences involves mere routine experimentation. Protector sequences can be readily identified by cloning viral library components into recombinant DNA expression vectors at positions such that the protector sequence will be present on the 5′ region of mRNA encoding a readily detectable substance such as β-gal or CAT. The recombinant DNA expression vector is then transfected into an eukaryotic host cell and the transformed host cell is then challenged with the same virus from which the protector sequence was isolated. Expression of the "protected" readily detectable substance (reporter gene product) in the recombinant DNA vector transformed and viral infected host cells is then assayed to determine the presence of a protector sequence.

Plasmid pUCLNβ-1 illustrates an eukaryotic expression vector comprising a protector sequence.Deletion of the ~440 bp of VSV cDNA from plasmid pUCLNβ-1 by PstI/BamHI digestion provides an eukaryotic expression vector of use in identifying other protector sequences. Other illustrative DNA expression vectors which would be useful in identifying protector sequence are well-known in the art. See Maniatis at 16.21. Vectors which contain a polycloning site at the 5′ of DNA sequences encoding a readily detectable substance are preferred vectors for isolating protector sequences. Other recombinant DNA expression vectors are also useful but may require the synthesis of DNA linkers to facilitate insertion of the protector sequences into an otherwise incompatible cloning site.

Eukaryotic expression vectors comprising viral-derived DNA sequences to be evaluated for protector activity are then introduced into host cells. The choice of host cells is limited only to those eukaryotic cells susceptible to infection by the virus from which the putative protector sequence was isolated. Following transfection the host cells comprising the recombinant DNA vectors comprising a viral DNA fragment so positioned as to result in its transcription on the 5′ end of a mRNA also encoding a readily detectable substance such as β-gal or CAT are infected with virus. Methods for the introduction of recombinant DNA vectors into eukaryotic cells are well-known in the art. See Maniatis at 16.30-16.55 and Example 9. Following introduction of the recombinant DNA vector comprising the viral protector sequence into eukaryotic host cells, the cells are screened to confirm the presence of the vector by assaying for the production of a readily detectable substance such as β-gal or CAT. Protocols for both β-gal and CAT assays are provided in Maniatis at 16.56-16.67. Eukaryotic host cells which assay as positive for the readily detectable substance (reporter gene products) are then challenged with the virus from which the protector sequences were isolated. The continuance of expression of the readily detectable substance during viral suppression of non-viral protein synthesis is the definitive criterion of a DNA sequence being a protector sequence.

The presence of an intact infective virus within the cell is undesirable and the need for which is circumvented by the cloning of the viral suppressor element and the subsequent introduction of the suppressor element into the host cell.

The mechanisms by which viruses suppress non-viral protein synthesis appear to be diverse and are only partially elucidated. An understanding of the mechanisms involved is however quite unnecessary to practice the full scope of the present invention. The underlying principle of the present invention is that viruses which suppress non-viral protein synthesis do so through production of molecules (protein and/or nucleic acids) which retard or prevent production of non-viral proteins. Viral suppression of non-viral protein synthesis occurs either by an inhibition of non-viral mRNA synthesis or by interfering with translation of the non-viral mRNA into proteins. The VSV N gene product mediates the suppression of non-viral protein synthesis seen in VSV infected cells. Thus, the VSV N gene provides a viral suppressor for purposes of the present invention.

The present invention provides a method for isolating "suppressors" from viruses which suppress non-viral protein sequences. Suppressor isolation merely involves screening the viral genomic library, which was prepared for isolation of protector sequences, for the ability of said genomic library components to suppress host cell protein synthesis, but not syntheses of a gene of interest containing a protector sequence. Skilled artisans realize that screening of viral genomic libraries for suppressor sequences requires only the expression of the suppressor gene product within the host cell and an analysis of protein synthesis by the host cell. Gene expression in eukaryotic cells is well-known in art of molecular biology. See Maniatis at 16.3-16.73. Rates of RNA synthesis are readily determinable by assaying the incorporation of radiolabelled uridine into RNA. Protein synthesis is readily monitored through incorporation of radiolabelled amino acids into protein. The isolation and identification of suppressor sequences merely requires the application of techniques well known in the art of molecular biology.

Thus, the present invention provides a method for isolating viral suppressor encoding sequences from double stranded DNA viruses which suppress non-viral protein synthesis consisting of:

(a) isolating the DNA from the virus, and

(b) preparing DNA fragments from the genomic DNA of step (a), and

(c) constructing recombinant DNA expression vectors comprising the DNA fragments of step (b) positioned 3' to an eukaryotic promoter so as to result in transcription of the DNA fragment of step (b), and

(d) transforming the recombinant DNA vector of step (c) into a host cell, and

(e) assaying the host cell of step (d) for suppression of non-viral protein synthesis, and

(f) isolating the suppressor sequences from the recombinant DNA vectors of step (c) which suppress non-viral protein synthesis.

Double stranded DNA viruses suitable for preparation of suppressor sequences include, but are not limited to vaccinia, herpes, equine abortion virus, and adenovirus. Suppressor sequences can also be prepared from RNA viruses including, but not limited to encephalomyocarditis virus, Newcastle disease virus, Sindbis, Sendai, VSV, mengovirus, and Venezuela equine encephalitis. The method for isolating viral suppressor encoding sequences from RNA viruses consists of:

(a) isolating the RNA from the virus, and

(b) preparing cDNA from the RNA of step (a),

(c) preparing cDNA fragments of the cDNA of step (b),

(d) constructing recombinant DNA expression vectors comprising the DNA fragments of step (c) positioned 3' to an eukaryotic promoter so as to result in transcription of the DNA fragment of step (c), and

(e) transforming the recombinant DNA vector of step (d) into a host cell, and

(f) assaying the host cell of step (e) for suppression of non-viral protein synthesis, and

(g) isolating the suppressor sequences from the recombinant DNA vectors of step (c) which suppress non-viral protein synthesis.

A further aspect of the vectors of the present invention is their utility as recombinant DNA expression vectors in prokaryotic host cells. Plasmids pUCLNβ-1, pUCLNβ-2, pUCLNβ-3, pUCLNβ-4, and pUCLNβ-5 were routinely grown in E. coli cells. During the course of the plasmid preparations it was noted that the E. coli host cells harboring the pUCLNβ-5 plasmid grew slowly and appeared atypical under light microscopic evaluation. Analysis of this unexpected result revealed that the host cells transformed with members of the pUCLNβ series were over-producing β-gal. Quantitation of β-gal levels in host cells transformed with the pUCLNβ series is presented in Table 4 below.

Table IV

Level of β-gal in E. coli Extracts Containing
pUCLNβ Plasmids

| Plasmid | Units/mg |
|---------|----------|
| None | 20 |
| pUCLNβ-1 | 1066 |
| pUCLNβ-2 | 173 |
| pUCLNβ-3 | 111 |
| pUCLNβ-4 | 25 |
| pUCLNβ-5 | 50788 |

The β-gal assay was performed in substantial accordance with the method of Casadaban, which was previously incorporated by reference. The data of Table IV indicate that the VSV-derived sequence functions as a translation initiation site. The lower levels of β-gal produced by plasmid pUCLNβ-4, which unlike the other members of the pUCLNβ series does not contain the plasmid pUC13-derived β-gal promoter, indicates that transcription may be initiating within the β-gal promoter while translation initiates within the VSV-derived sequence. Gel electrophoresis of the E. coli extracts revealed that the N gene/β-gal hybrid protein had a molecular weight corresponding to translation arising within the VSV-derived sequence. For purposes of expressing a product of interest in prokaryotic cells derivatives of plasmids pUCLNβ-1, pUCLNβ-2, pUCLNβ-3, and pUCLNβ-5 are preferred while plasmids pUCLNβ-1, and pUCLNβ-5 are more preferred and plasmid pUCLNβ-5 is especially preferred. β-gal functioned in the aforementioned plasmids as a reporter gene encoding a readily detectable substance. Skilled artisans realize that any gene encoding a polypeptide product of interest when inserted in proper reading frame 3′ to the VSV-derived sequence would be expressed as a fusion product with the truncated N gene in a manner analogous to the N gene/β-gal hybrid protein produced by the pUCLNβ series. Skilled artisans also realize that it is common in the art to express polypeptide products of interest as fusion products (hybrid proteins). Numerous methods are available to cleave the polypeptide product of interest from the undesired portion of the hybrid protein. The synthesis of DNA linkers is commonly used to insert an appropriate cleavage site at the junction of the two sequences comprising the hybrid protein encoding sequence to produce a "cleavage-linker", which allows a facile separation of the components of the hybrid protein. For example, Asp-Asp-Asp-Asp-Lys is an uncommon amino acid sequence which is cleaved by bovine enterokinase. Likewise, CNBr can be used to cleave polypeptides at methionine residues. Similarly, kallekriens are routinely utilized to cleave polypeptides at the occurrence of a pair of basic amino acids (Lys, Arg, or His). DNA sequences encoding the illustrative "cleavage linkers" can be easily synthesized by techniques well known in the art. The incorporation of "cleavage linkers" into the vectors of the present invention adds to the general utility of the vectors and thus is a preferred embodiment of the invention as it relates to expression of polypeptide products of interest in both eukaryotic and prokaryotic cells.

The term polypeptide products of interest includes polypeptide products useful for medical, commercial, or veterinary purposes. Examples of polypeptide products of interest include, but are not limited to human insulin, human pro-insulin, human insulin A chain, human insulin B chain, human growth hormone, porcine growth hormone, insulin-like growth factor I, insulin-like growth factor II, human interferon $\alpha$, human interferon $\beta$, human interferon $\gamma$, tissue-plasminogen activator, viral antigens, interleukin (IL)-1, IL-2, IL-3, IL-4, IL-5, IL-6, human erythropoetin, human granulocyte macrophage colony stimulating factor human protein C, human protein S, human thrombomodulin and human transferrin.

Example 1

Preparation of BK Virus DNA

BK virus is obtained from the American Type Culture Collection under the accession number ATCC VR-837. The virus is delivered in freeze-dried form and resuspended in Hank's balanced salts (Gibco, 3175 Staley Road, Grand Island, NY 14072) to a titer of about $10^5$ plaque-forming units (pfu)/ml. The host cell of choice for the preparation of BK virus DNA is the Vero cell-line, which can be obtained from the American Tissue Culture Collection, 12301 Parklawn Dr., Rockville, MD 20852, under the accession number, ATCC CCL81.

About five 75 mm$^2$ polystyrene flasks comprising confluent monolayers of about $10^6$ Vero cells are used to prepare the virus. About 1 ml of BK virus at a titer of $10^5$ pfu/ml is added to each flask, which is then incubated at 37°C for one hour, and then, fresh culture medium (Dulbecco's Modified Eagle's Medium, Gibco, supplemented with 10% fetal bovine serum) is added, and the infected cells are incubated at 37°C for 10-14 days or until the full cytopathogenic effect of the virus is noted. This cytopathogenic effect varies from cell line to cell line and from virus to virus but usually consists of cells rounding up, clumping, and sloughing off the culture disk.

The virus is released from the cells by three freeze-thaw cycles, and the cellular debris is removed by centrifugation at 5000Xg. The virus in 1 liter of supernatant fluid is precipitated and collected by the addition of 100 g of PEG-6000, incubation of the solution for 24 hours at 4°C, and centrifugation at 5000Xg for 20 minutes. The pellet is dissolved in 0.1X SSC buffer (IXSSC = 0.15 M NaCl and 0.015 M sodium citrate, pH = 7) at 1/100th of the original volume. The virus suspension is layered onto a 15 ml solution of saturated KBr in a tube, which is centrifuged at 75,000Xg for 3 hours. Two bands are evident in the KBr solution after centrifugation. The lower band, which contains the complete virion, is collected and desalted on a Sephadex® G-50 column (Sigma Chemical Co., P.O. Box 14508, St. Louis, MO 63178) using TE (10 mM Tris-HCl, pH = 7.8, and 1 mM EDTA) as an elution buffer.

Sodium dodecyl sulfate (SDS) is added to the solution of purified virions to a concentration of 1%; pronase is added to a concentration of 100 μg/ml, and the solution is incubated at 37°C for 2 hours. Cesium chloride is then added to the solution to a density of 1.56 g/ml, and ethidium bromide is added to the solution to a final concentration of 100 μg/ml. The solution is centrifuged in a Sorvall (DuPont Inst. Products, Biomedical Division, Newton, CT 06470) 865 rotor or similar vertical rotor at 260,000Xg for 24 hours. After centrifugation, the band of virus DNA is isolated and extracted five times with isoamyl alcohol saturated with 100 mM Tris-HCl, pH = 7.8. The solution of BK virus DNA is then dialyzed against TE buffer until the 260 nm/280 nm absorbance ratio of the DNA is between 1.75 and 1.90. The DNA is precipitated by adjusting the NaCl concentration to 0.15 M, adding two volumes of ethanol, incubating the solution at -70°C for at least 2 hours, and centrifuging the solution at 12,000Xg for 10 minutes. The resulting pellet of BK virus DNA is suspended in TE buffer at a concentration of 1 mg/ml.

Example 2

Construction of Intermediate Plasmids pBKE1 and pBKE2

About one µg of the BK virus DNA prepared in Example 1 in one µl of TE buffer was dissolved in 2 µl of 10X EcoRI buffer (1.0 M Tris-HCl, pH = 7.5; 0.5 M NaCl; 50 mM MgCl$_2$; and 1 mg/ml bovine serum albumin (BSA)) and 15 µl of H$_2$O. About 2 µl (~10 units; all enzyme units referred to herein, unless otherwise indicated, refer to the unit definitions of New England Biolabs, 32 Tozer Road, Beverly, MA 01915-9990, although the actual source of the enzymes may have been different) of restriction enzyme EcoRI were added to the solution of DNA, and the resulting reaction was incubated at 37°C for two hours.

About 1 µg of plasmid pUC8 (available from Pharmacia P-L Biochemicals, 800 Centennial Ave., Piscataway, N.J. 08854) in 1 µl of TE buffer was digested with EcoRI in substantial accordance with the procedure used to prepare the EcoRI-digested BK virus DNA. The EcoRI-digested plasmid pUC8 DNA was diluted to 100 µl in TE buffer; ~0.06 units of calf-intestinal alkaline phosphatase were added to the solution, and the resulting reaction was incubated at 37°C for 30 minutes. The solution was adjusted to contain 1X SET (5 mM Tris-HCl, pH = 7.8; 5 mM EDTA; and 150 mM NaCl), 0.3M NaOAc, and 0.5% SDS and then incubated at 65°C for 45 minutes. The phosphatase treatment prevents the pUC8 DNA from self ligating.

The EcoRI-digested BK virus and plasmid pUC8 DNA were extracted first with buffered phenol and then with chloroform. The DNA was collected by adjusting the NaCl concentration of each DNA solution to 0.25 M, adding two volumes of ethanol, incubating the resulting mixtures in a dry ice-ethanol bath for 5 minutes, and centrifuging to pellet the DNA. The supernatants were discarded, and the DNA pellets were rinsed with 70% ethanol, dried, and resuspended in 10 µl and 30 µl of TE buffer for the BK and plasmid pUC8 samples, respectively.

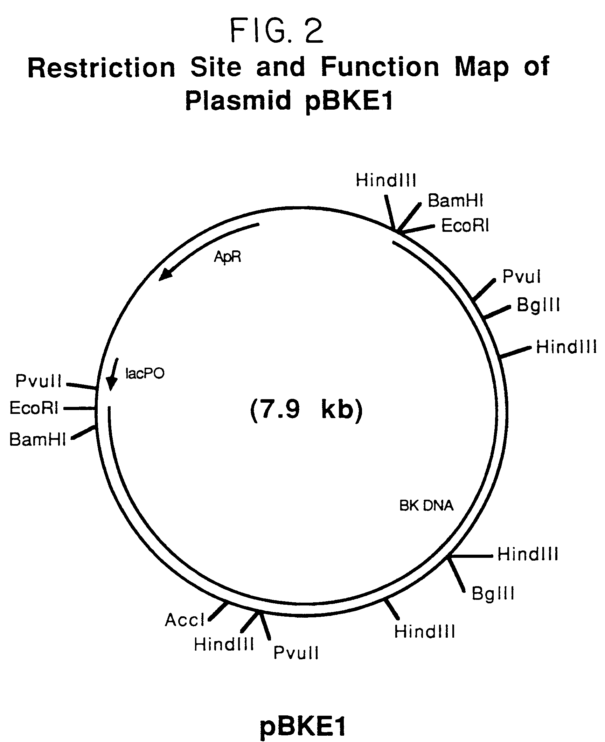

About 3 µl of H$_2$O and 1 µl of 10X ligase buffer (0.5 M Tris-HCl, pH = 7.8; 100 mM MgCl$_2$; 200 mM DTT; 10 mM ATP; and 0.5 mg/ml BSA) were added to a mixture of 2 µl of the EcoRI-digested BK virus and 1 µl of the EcoRI-digested plasmid pUC8 DNA. One µl (~1000 units) of T4 DNA ligase were added to the solution of DNA, and the resulting reaction was incubated at 16°C overnight. The ligated DNA constituted the desired plasmids pBKE1 and pBKE2, which differ only with respect to the orientation of the inserted BK virus DNA. A restriction site and function map of plasmid pBKE1 is presented in Figure 2 of the accompanying drawings.

A 50 ml culture of E. coli K12 HB101, available from Pharmacia P-L Biochemicals, in L broth was grown to an optical density at 650 nanometers (O.D.$_{650}$) of approximately 0.4 absorbance units. L broth is also known as LB Medium. L broth consists of Bacto-tryptone 10g/l, Bacto-yeast extract 5g/l and NaCl 10g/l, pH 7.5. The culture was chilled on ice for ten minutes, and the cells were collected by centrifugation. The cell pellet was resuspended in 25 ml of cold 100 mM MgCl$_2$ and incubated on ice for 25 minutes. The cells were once again pelleted by centrifugation, and the pellet was resuspended in 2.5 ml of cold 100 mM CaCl$_2$ and incubated for 30 minutes on ice. After the incubation, the cells are competent for the uptake of transforming DNA.

Two hundred µl of this cell suspension were mixed with the ligated DNA prepared above and incubated on ice for 30 minutes. At the end of this period, the cells were placed in a water bath at 42°C for 2 minutes and then returned to the ice for an additional 10 minutes. The cells were collected by centrifugation and resuspended in one ml of L broth and incubated at 37°C for 1 hour.

Aliquots of the cell mixture were plated on L-agar (L broth with 15 grams of agar per liter) plates containing 100 µg ampicillin/ml, 40 µg 5-bromo-4-chloro-3-indolyl-β-D-galactosidase (X-gal)/ml, and 40 µg isopropylthio-β-D-galactosidase (IPTG)/ml. The plates were incubated at 37°C overnight. Colonies that contain a plasmid without an insert, such as E. coli K12 HB101/pUC8, appear blue on these plates. Colonies that contain a plasmid with an insert, such as E. coli K12 HB101/pBKE1, are white. Several white colonies were selected and screened by restriction enzyme analysis of their plasmid DNA for the presence of the ~5.2 kb EcoRI restriction fragment of BK virus. Plasmid DNA was obtained from the E. coli K12 HB101/pBKE1 and E. coli K12 HB101/pBKE2 cells in substantial accordance with the procedure for isolating plasmid DNA that is described in the following Example, although the procedure is done on a smaller scale, and the CsCl gradient steps are omitted, when the plasmid DNA is isolated only for restriction enzyme analysis.

Example 3

Construction of Intermediate Plasmids pBKneo1 and pBKneo2

E. coli K12 HB101/pdBPV-MMTneo cells are obtained in lyophil form from the American Type Culture Collection under the accession number ATCC 37224. The lyophilized cells are plated on L-agar plates containing 100 µg/ml ampicillin and incubated at 37°C to obtain single colony isolates.

One liter of L broth (10 g tryptone, 10 g NaCl, and 5 g yeast extract per liter) containing 50 µg/ml ampicillin was inoculated with a colony of E. coli K12 HB101/pdBPV-MMTneo and incubated in an air-shaker at 37°C until the O.D.$_{590}$ was ~1 absorbance unit, at which time 150 mg of chloramphenicol were added to the culture. The incubation was continued for about 16 hours; the chloramphenicol addition inhibits protein synthesis, and thus inhibits further cell division, but allows plasmid replication to continue.

The culture was centrifuged in a Sorvall GSA rotor (DuPont Co., Instrument Products, Biomedical Division, Newtown, CN 06470) at 6000 rpm for 5 minutes at 4°C. The resulting supernatant was discarded, and the cell pellet was washed in 40 ml of TES buffer (10 mM Tris-HCl, pH = 7.5; 10 mM NaCl; and 1 mM EDTA) and then repelleted. The supernatant was discarded, and the cell pellet was frozen in a dry ice-ethanol bath and then thawed. The thawed cell pellet was resuspended in 10 ml of a solution of 25% sucrose and 50 mM EDTA. About 1 ml of a 5 mg/ml lysozyme solution; 3 ml of 0.25 M EDTA, pH = 8.0; and 100 µl of 10 mg/ml RNAse A were added to the solution, which was then incubated on ice for 15 minutes. Three ml of lysing solution (prepared by mixing 3 ml 10% Triton X-100; 75 ml of 0.25 M EDTA, pH = 8.0; 15 ml of 1 M Tris-HCl, pH = 8.0; and 7 ml of water) were added to the lysozyme-treated cells, mixed, and the resulting solution incubated on ice for another 15 minutes. The lysed cells were frozen in a dry ice-ethanol bath and then thawed.

The cellular debris was removed from the solution by centrifugation at 25,000 rpm for 40 minutes in an SW27 rotor (Beckman, 7360 N. Lincoln Ave., Lincolnwood, IL 60646) and by extraction with buffered phenol. About 30.44 g of CsCl and ~1 ml of a 5 mg/ml ethidium bromide solution were added to the cell extract, and then, the volume of the solution was adjusted to 40 ml with TES buffer. The solution was decanted into a VTi50 ultra-centrifuge tube (Beckman), which was then sealed and centrifuged in a VTi50 rotor at 42,000 rpm for ~16 hours. The resulting plasmid band, visualized with ultraviolet light, was isolated and then placed in a Ti75 tube and rotor (Beckman) and centrifuged at 50,000 rpm for 16 hours. Any necessary volume adjustments were made using TES containing 0.761 g/ml CsCl. The plasmid band was again isolated, extracted with salt-saturated isopropanol to remove the ethidium bromide, and diluted 1:3 with TES buffer. Two volumes of ethanol were then added to the solution, which was then incubated overnight at -20°C. The plasmid DNA was pelleted by centrifuging the solution in an SS34 rotor (Sorvall) for 15 minutes at 10,000 rpm.

The ~1 mg of plasmid pBdPV-MMTneo DNA obtained by this procedure was suspended in 1 ml of TE buffer and stored at -20°C. The foregoing plasmid isolation procedure is generally used when large amounts of very pure plasmid DNA are desired. The procedure can be modified to increase the speed of DNA isolation when the purity of the DNA is not critical. For example, when transformants are being screened for the presence of a given plasmid, only about 5 ml of cultured cells are used; the cells are lysed in an appropriately scaled-down amount of lysis buffer, and the centrifugation steps are replaced with phenol and chloroform extractions.

About 5 µg (5 µl) of the plasmid pdBPV-MMTneo DNA prepared above and five µg (5 µl) of the BK virus DNA prepared in Example 1 were each digested at 37°C for 2 hours in a solution containing 2 µl of 10X BamHI buffer (1.5 M NaCl; 60 mM Tris-HCl, pH = 7.9; 60 mM MgCl$_2$; and 1 mg/ml BSA), 1 µl of restriction enzyme BamHI, and 7 µl of H$_2$O. The reaction was stopped by an extraction with an equal volume of phenol, followed by two extractions with chloroform. Each BamHI-digested DNA was then precipitated, collected by centrifugation, and resuspended in 5 µl of H$_2$O.

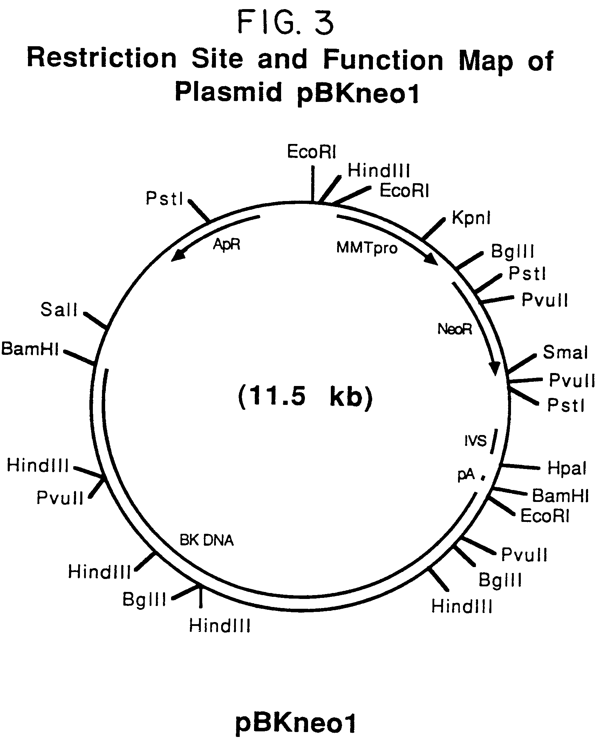

About 1 µl of 10X ligase buffer was added to a mixture of BamHI-digested plasmid pdBPV-MMTneo (1 µl) and BamHI-digested BK virus DNA (1 µl). After 1 µl (~1000 units) of T4 DNA ligase and 6 µl of H$_2$O were added to the mixture of DNA, the resulting reaction was incubated at 16°C overnight. The ligated DNA constituted the desired plasmids pBKneol and pBKneo2, which differ only with respect to the orientation of the BK virus DNA. A restriction site and function map of plasmid pBKneol is presented in Figure 3 of the accompanying drawings.

E. coli K12 HB101 cells are available in lyophilized form from the Northern Regional Research Laboratory under the accession number NRRL B-15626. E. coli K12 HB101 cells were cultured, made competent for transformation, and transformed with the ligated DNA prepared above in substantial accordance with the procedure of Example 2. The transformed cells were plated on L-agar plates containing 100 µg/ml ampicillin. E. coli K12 HB101/pBKneo1 and E. coli K12/pBKneo2 transformants were identified by their ampicillin-resistant phenotype and by restriction enzyme analysis of their plasmid DNA.

Example 4

Construction of Intermediate Plasmid pBLcat

A. Construction of Intermediate Plasmid pLPcat

The virion DNA of adenovirus 2 (Ad2) is a double-stranded linear molecule about 35.94 kb in size. The Ad2 late promoter is isolated on an ~0.32 kb AccI-PvuII restriction fragment of the Ad2 genome; this ~0.32 kb restriction fragment corresponds to the sequence between nucleotide positions 5755 and 6071 of the Ad2 genome. To isolate the desired ~0.32 kb AccI-PvuII restriction fragment, Ad2 DNA is first digested with restriction enzyme BalI, and the ~2.4 kb BalI restriction fragment that comprises the entire sequence of the ~0.32 kb AccI-PvuII restriction fragment is isolated. Then, the ~2.4 kb BalI restriction fragment is digested with AccI and PvuII to obtain the desired fragment.

About 50 μg of Ad2 DNA (available from BRL) are dissolved in 80 μl of $H_2O$ and 10 μl of 10X BalI buffer (100 mM Tris-HCl, pH = 7.6; 120 mM $MgCl_2$; 100 mM DTT; and 1 mg/ml BSA). About 10 μl (~20 units) of restriction enzyme BalI are added to the solution of Ad2 DNA, and the resulting reaction is incubated at 37°C for 4 hours.

The BalI-digested DNA is loaded onto an agarose gel and separated by electrophoresis. Visualization of the separated DNA is accomplished by staining the gel in a dilute solution (0.5 μg/ml) of ethidium bromide and exposing the stained gel to long-wave ultraviolet (UV) light. One method to isolate DNA from agarose is as follows. A small slit is made in the gel in front of the desired fragment, and a small piece of NA-45 DEAE membrane (Schleicher and Schuell, Keene, NH 03431) is placed in each slit. Upon further electrophoresis, the DNA non-covalently binds to the DEAE membrane. After the desired fragment is bound to the DEAE membrane, the membrane is removed and rinsed with low-salt buffer (100 mM KCl; 0.1 mM EDTA; and 20 mM Tris-HCl, pH = 8). Next, the membrane is placed in a small tube and immersed in high-salt buffer (1 M NaCl; 0.1 mM EDTA; and 20 mM Tris-HCl, pH = 8) and then incubated at 65°C for one hour to remove the DNA from the DEAE paper. After the 65°C incubation, the incubation buffer is collected and the membrane rinsed with high-salt buffer. The high-salt rinse solution is pooled with the high-salt incubation buffer.

The volume of the high salt-DNA solution is adjusted so that the NaCl concentration is 0.25 M, and then three volumes of cold, absolute ethanol are added to the solution. The resulting solution is mixed and placed at -70°C for 10-20 minutes. The solution is then centrifuged at 15,000 rpm for 15 minutes. After another precipitation to remove residual salt, the DNA pellet is rinsed with ethanol, dried, resuspended in 20 μl of TE buffer, and constitutes about 3 μg of the desired restriction fragment of Ad2. The purified fragment obtained is dissolved in 10 μl of TE buffer.

About 6 μl of $H_2O$ and 2 μl of 10X AccI buffer (60 mM NaCl; 60 mM Tris-HCl, pH = 7.5; 60 mM $MgCl_2$; 60 mM DTT; and 1 mg/ml BSA) are added to the solution of the ~2.4 kb BalI restriction fragment of Ad2. After the addition of about 2 μl (~10 units) of restriction enzyme AccI to the solution of DNA, the reaction is incubated at 37°C for 2 hours. After the AccI digestion, the DNA is collected by ethanol precipitation and resuspended in 16 μl of $H_2O$ and 2 μl of 10X PvuII buffer (600 mM NaCl; 60 mM Tris-HCl, pH = 7.5; 60 mM $MgCl_2$; 60 mM DTT; and 1 mg/ml BSA). After the addition of about 2 μl (about 10 units) of restriction enzyme PvuII to the solution of DNA, the reaction is incubated at 37°C for 2 hours.

The AccI-PvuII-digested, ~2.4 kb BalI restriction fragment of Ad2 is loaded onto an ~6% polyacrylamide gel and electrophoresed until the ~0.32 kb AccI-PvuII restriction fragment that comprises the Ad2 late promoter is separated from the other digestion products. The gel is stained with ethidium bromide and viewed using UV light, and the segment of gel containing the ~0.32 kb AccI-PvuII restriction fragment is cut from the gel, crushed, and soaked overnight at room temperature in ~250 μl of extraction buffer (500 mM $NH_4OAc$; 10 mM MgOAc; 1 mM EDTA; and 0.1% SDS). The following morning, the mixture is centrifuged, and the pellet is discarded. The DNA in the supernatant is precipitated with ethanol; about 2 μg of mRNA are added to ensure complete precipitation of the desired fragment. About 0.2 μg of the ~0.32 kb AccI-PvuII restriction fragment are obtained and suspended in 7 μl of $H_2O$.

About 0.25 μg (in 0.5 μl) of BclI linkers (5'-CTGATCAG-3', available from New England Biolabs), which had been kinased was added to the solution of the ~0.32 kb AccI-PvuII restriction fragment, and then, 1 μl (~1000 units) of T4 DNA ligase and 1 μl of 10X ligase buffer were added to the solution of DNA, and the resulting reaction was incubated at 16°C overnight. Bacteriophage T4 polynucleotide (DNA) kinase and its applicability to small oligonucleotides is described in Maniatis at 5.60-5.71. The BclI linkers could only ligate to the PvuII end of the AccI-PvuII restriction fragment. DNA sequencing later revealed that four BclI linkers attached to the PvuII end of the AccI-PvuII restriction fragment. These extra BclI linkers can be removed by BclI digestion and religation; however, the extra BclI linkers were not removed as the linkers do not interfere with the proper functioning of the vectors that comprise the extra linkers.

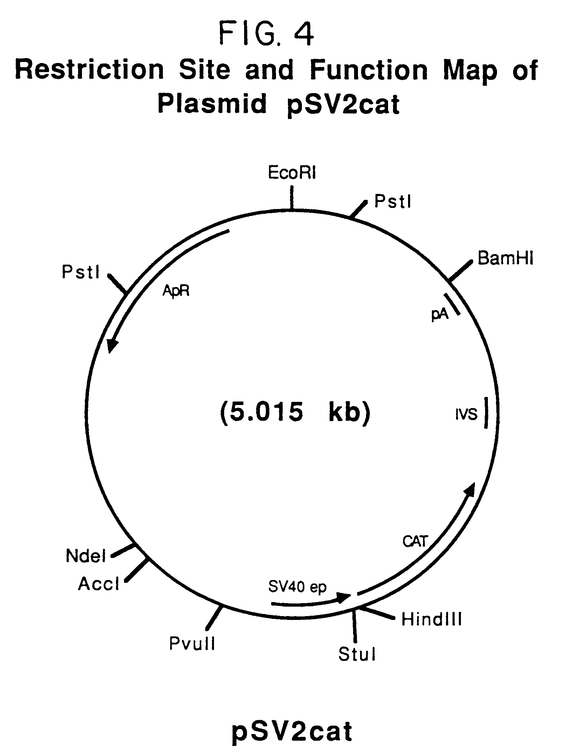

E. coli K12 HB101/pSV2cat cells are obtained in lyophilized form from the ATCC under the accession number ATCC 37155, and plasmid pSV2cat DNA was isolated from the cells in substantial accordance with the procedure of Example 3. A restriction site and function map of plasmid pSV2cat is presented in Figure 4 of the accompanying drawings. About 1 mg of plasmid pSV2cat DNA is obtained and dissolved in 1 ml of TE buffer. About 3 μg (3 μl) of the plasmid pSV2cat DNA were added to 2 μl of 10X AccI buffer and 16 μl of H$_2$O, and then, 3 μl (about 9 units) of restriction enzyme AccI were added to the solution of pSV2cat DNA, and the resulting reaction was incubated at 37°C for 2 hours. The AccI-digested plasmid pSV2cat DNA was then digested with restriction enzyme StuI by adding 3 μl of 10X StuI buffer (1.0M NaCl; 100 mM Tris-HCl, pH = 8.0; 100 mM MgCl$_2$; 60 mM DTT; and 1 mg/ml BSA), 5 μl of H$_2$O, and about 2 μl (about 10 units) of restriction enzyme StuI. The resulting reaction was incubated at 37°C for 2 hours. The reaction was terminated by extracting the reaction mixture once with phenol, then twice with chloroform. About 0.5 μg of the desired fragment was obtained and dissolved in 20 μl of TE buffer.

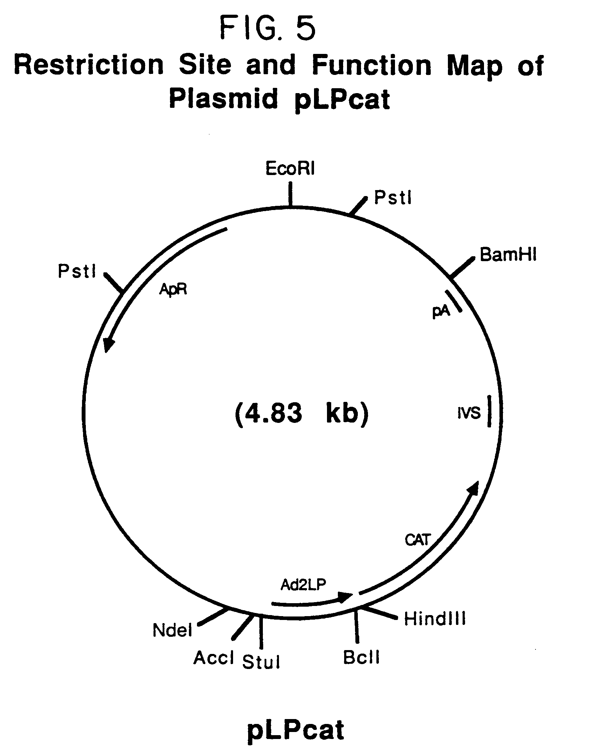

About 4 μl of the AccI-StuI-digested plasmid pSV2cat DNA were mixed with about 7 μl of the ~0.32 kb AccI-PvuII (with BclI linkers attached) restriction fragment of Ad2, and after the addition of 3 μl of 10X ligase buffer, 15 μl of H$_2$O, and 2 μl (about 1000 units) of T4 DNA ligase, the ligation reaction was incubated at 16°C overnight. The ligated DNA constituted the desired plasmid pLPcat, a plasmid that comprises the Ad2 late promoter positioned so as to drive transcription, and thus expression, of the chloramphenicol acetyltransferase gene. A restriction site and function map of plasmid pLPcat is presented in Figure 5 of the accompanying drawings.

The ligated DNA was used to transform E. coli K12 HB101 cells in substantial accordance with the procedure of Example 3. The transformed cells were plated on L agar containing 50 μg/ml ampicillin; restriction enzyme analysis of plasmid DNA was used to identify the E. coli K12 HB101/pLPcat transformants. Plasmid pLPcat DNA was isolated from the transformants for use in subsequent constructions in substantial accordance with the plasmid isolation procedure described in Example 3.

B. Final Construction of Plasmid pBLcat

About 88 μg of plasmid pBKneo1 DNA in 50 μl of TE buffer were added to 7.5 μl of 10X AccI buffer, 30 μl of H$_2$O, and 15 μl (about 75 units) of restriction enzyme AccI, and the resulting reaction was incubated at 37°C for 2 hours. The AccI-digested BK virus DNA was loaded on an agarose gel, and the ~1.4 kb fragment that contains the BK enhancer was separated from the other digestion products. The ~1.4 kb AccI restriction fragment was then isolated in substantial accordance with the procedure described in Example 4A. About 5 μg of the fragment were resuspended in 5 μl of 10X PvuII buffer, 45 μl of H$_2$O, and 5 μl (about 25 units) of restriction enzyme PvuII, and the resulting reaction was incubated at 37°C for 2 hours. The PvuII-digested DNA was then isolated and prepared for ligation in substantial accordance with the procedure of Example 4A. About 2 μg of the desired ~1.28 kb AccI-PvuII fragment were obtained and dissolved in 5 μl of TE buffer.

About 1 μg of plasmid pLPcat DNA was dissolved in 5 μl of 10X AccI buffer and 40 μl of H$_2$O. About 5 μl (~25 units) of restriction enzyme AccI were added to the solution of plasmid pLPcat DNA, and the resulting reaction was incubated at 37°C. The AccI-digested plasmid pLPcat DNA was precipitated with ethanol and resuspended in 5 μl of 10X StuI buffer, 40 μl of H$_2$O, and 5 μl (about 25 units) of restriction enzyme StuI, and the resulting reaction was incubated at 37°C for 2 hours. The AccI-StuI-digested plasmid pLPcat DNA was precipitated with ethanol several times to purify the ~4.81 kb AccI-StuI restriction fragment that comprises the E. coli origin of replication and Ad2 late promoter away from the other digestion product, a restriction fragment about 16 bp in size. About 1 μg of the desired ~4.81 kb restriction fragment was obtained and dissolved in 20 μl of TE buffer.

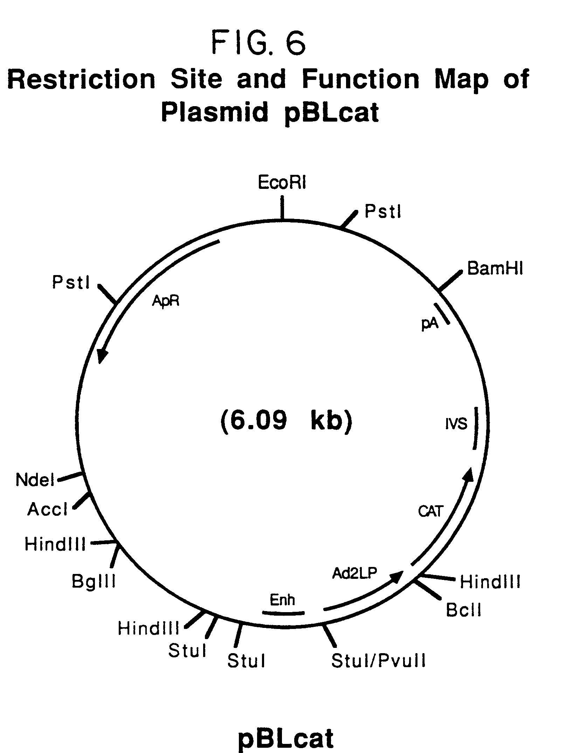

The 5 μl of ~4.81 kb AccI-StuI restriction fragment of plasmid pLPcat were added to 5 μl of ~1.28 kb AccI-PvuII restriction fragment of BK virus. After the addition of 3 μl of 10X ligase buffer, 15 μl of H$_2$O, and 2 μl (about 1000 units) of T4 DNA ligase to the mixture of DNA, the resulting ligation reaction was incubated at 16°C overnight. The ligated DNA constituted the desired plasmid pBLcat. A restriction site and function map of plasmid pBLcat is presented in Figure 6 of the accompanying drawings.

The ligated DNA was used to transform E. coli K12 HB101 cells in substantial accordance with the procedure described in Example 3. E. coli K12 HB101/pBLcat transformants were identified by restriction enzyme analysis of their plasmid DNA. Plasmid pBLcat DNA was prepared for use in subsequent constructions in substantial accordance with the procedure of Example 3.

Example 5

Plasmids pUCLNβ-2 and pUCLNβ-3

A. Preparation of Plasmid pUCLNβ-1

Plasmid pUCLNβ-1 is obtained in lyophil form from the Northern Regional Research Laboratory under the accession number NRRL B-18715, deposited September 21, 1990. Plasmid pUCLNβ-1 was constructed by cloning a 440 bp cDNA VSV sequence comprising the leader and N gene into plasmid pUC13 followed by addition of a β-galactosidase gene at the 3' end of the VSV sequence. A restriction site and function map of plasmid pUCLNβ-1 is provided in Figure 7. Plasmid pUCLNβ-1 comprises the ampicillin gene and origin of replication of plasmid pUC13.

The lyophilized cells are plated on L agar plates containing 100 μg/ml ampicillin and incubated at 37°C to obtain single colony isolates. One liter of L broth (10 g tryptone, 10 g NaCl, and 5 g yeast extract per liter) was inoculated with a colony of E. coli K12 HB101/pUCLNβ-1 and incubated at 37°C for approximately 18 hours.

Plasmid pUCLNβ-1 was then isolated in substantial accordance with the method of Example 3.

B. Construction of Plasmids pUCLNβ-2 and pUCLNβ-3

Approximately 250 ng of plasmid pUCLNβ-1 was digested with ~10 U of HindIII for ~1 hour in a total volume of 20μl. HindIII was purchased from New England Biolabs. The HindIII digestion buffer consists of 50 mM NaCl, 50 mM Tris-HCl (pH = 8.0), 10 mM MgCl$_2$, and 100 μg/ml bovine serum albumin. The HindIII digested DNA was then extracted with an equal volume of phenol, followed by two extractions with chloroform. The HindIII-digested DNA was then precipitated with 70% ethanol and centrifuged at 10,000 X g for 10 min. The ethanol was then evaporated under vacuum. The HindIII-digested DNA was dissolved in a minimal amount of 10 mM Tris-HCl (pH = 8.0) and subjected to calf intestinal phosphatase (CIP) to prevent self-ligation. Calf intestinal phosphatase (Type XX) was obtained from Sigma Chemical Company, P.O. Box 14508, St. Louis, MO. 63178. The dephosphorylation reaction consisted of 5 μl of 10X CIP buffer (0.5 M Tris-HCl (pH = 9.0), 10 mM MgCl$_2$, 1 mM ZnCl$_2$, 10 mM spermidine) ~5 μl of HindIII digested pUCLNβ-1 in 10 mM Tris-HCl, pH = 8.0; H$_2$O to 48 μl; and 2 μl of CIP (Sigma #P9517). After 30 minutes at 37°C an additional 2 μl of CIP was added and the 37°C incubation was continued for 30 minutes. ~40 μl of H$_2$O, 10 μl of 10X STE (100 mM Tris-HCl (pH = 8.0), 1 M NaCl, 10 mM EDTA) and ~5 μl of 10% sodium dodecyl sulfate (SDS) was added. The mixture was then heated to 68°C for 15 minutes. The dephosphorylated HindIII digested plasmid pUCLNβ-1 DNA was extracted twice with phenol chloroform and twice with chloroform. The extracted DNA was then ethanol precipitated, dried under vacuum and resuspended in ~ 50 μl of TE (10mM Tris, pH = 7.4, 1mM EDTA).

Plasmid pBL-CAT was taught in Example 4B. The ~0.87 kb HindIII fragment of plasmid pBL-CAT was isolated by HindIII digestion followed by agarose gel electrophoresis. HindIII digestion proceeded in substantial accordance with the pUCLNβ-1 digestion taught previously. The ~0.87 kb HindIII fragment was harvested from the gel, extracted, precipitated, and resuspended in TE in substantial accordance with the teaching of Example 4B. Approximately 50 ng of the ~0.87 kb HindIII fragment of plasmid pBL-CAT and approximately 250 ng of the ~6.82 kb HindIII digested plasmid pUCLNβ-1 DNA were ligated using T4 ligase. The T4 DNA ligase was obtained from New England Biolabs. The ligation was performed in substantial accordance with the method set forth in Example 2. The ligation mixture was then used to transform E. coli K12 HB101 cells in substantial accordance with the teachings of Example 2. Transformants were selected on L-agar containing 100 μg/ml ampicillin. Plasmid DNA was harvested from the ampicillin resistant transformants in substantial accordance with the teachings of Example 3. Restriction endonuclease mapping of plasmid DNA was used to determine the orientation of the ~0.87 kb fragment of plasmid pBL-CAT. Plasmid pUCLNβ-2 contains the BL cassette, which resides on the ~0.87 kb HindIII fragment of plasmid pBL-CAT. In plasmid pUCLNβ-2 the BK enhancer and Adenovirus type II late promoter drive high level expression of the truncated N gene/ β-galactosidase fusion protein. A restriction site and function map of plasmid pUCLNβ-2 is provided in Figure 8. When the ~0.87 plasmid pBL-CAT-derived fragment is in the alternative orientation (plasmid pUCLNβ-3), the N gene/β-galactosidase message is driven from the BK promoter.

Example 6

Construction of Plasmid pUCLNβ-5

The ~373 bp AvrII fragment of plasmid pUCLNβ-2 was excised to generate plasmid pUCLNβ-5. Plasmid pUCLNβ-2 DNA was prepared as taught in Example 5. Approximately 250 ng of plasmid pUCLNβ-2 was digested with 10U of AvrII at 37°C for 1 hour in a 20 µl final volume comprising 50 mM NaCl, 10 mM Tris-HCl (pH = 7.4), 10 mM MgCl₂, 10 mM 2-mercaptoethanol, and 100 µg/ml bovine serum albumin. The AvrII digested plasmid pUCLNβ-2 DNA was separated by electrophoresis on agarose gel and the ~7.3 kb fragment was harvested in substantial accordance with the teaching of Example 4A. The ~7.3 kb AvrII fragment of plasmid pUCLNβ-2-was then ligated using T4 DNA ligase (New England Biolabs) to generate plasmid pUCLNβ-5.

The ~373 bp AvrII fragment of plasmid pUCLNβ-2 contains the Adenovirus type II late promoter. Thus, plasmid pUCLNβ-5 does not contain the Adenovirus type II late promoter, and the BK early promoter is improperly oriented for expression of the N gene β-galactosidase fusion protein. Preparation of plasmid pUCLNβ-5 DNA via transformation of E. coli K12 HB101 and the subsequent plasmid isolation was accomplished in substantial accordance with the teaching of Example 3. A restriction site and function map of pUCLNβ-5 is provided in Figure 9.

### Example 7

### Construction of Plasmid pUCLNβ-4

Plasmid pUCLNβ-4 was constructed by ligating the ~2122 bp ScaI/HindIII fragment of plasmid pSV2-CAT to the ~5021 bp ScaI/HindIII fragment of plasmid pUCLNβ-1.

Plasmid pSV2-CAT was used in the construction of plasmid pLP-CAT (Example 4). The plasmid pSV2-CAT DNA was prepared in substantial accordance with the teaching of Example 4. Plasmid pUCLNβ-1 DNA was prepared as taught in Example 5.

Approximately 250 ng of plasmid pUCLNβ-1 DNA was digested with 10 U of HindIII for 1 hour at 37°C in a 20 µl total volume of HindIII digestion buffer. The digest was extracted and the DNA was then precipitated with ethanol. After evaporation of the ethanol under vacuum, the HindIII digested plasmid pUCLNβ-1 DNA was digested with 10 U of ScaI (New England Biolabs) for 1 hour at 37°C in a 20 µl total digestion volume comprising 100 mM NaCL, 10 mM Tris-HCl (pH = 7.4), 10 mM MgCl₂, 1 mM dithiothreitol, and 100 µg/ml bovine serum albumin. The doubly digested pUCLNβ-1 DNA was then electrophoresed through an agarose gel and the ~5021 bp fragment was harvested as taught in Example 4B.

Approximately 100 ng of plasmid pSV2-CAT was sequentially digested with HindIII and ScaI in substantial accordance with the method taught above for plasmid pUCLNβ-1. The ~2122 bp fragment was then purified on an agarose gel and harvested as taught in Example 4B.

The ~5021 bp HindIII/ScaI fragment of plasmid pUCLNβ-1 and the ~2122 bp HindIII/ScaI fragment of plasmid pSV2-CAT were ligated in substantial accordance with the teaching of Example 5. A restriction site and function map of plasmid pUCLNβ-4 is provided in Figure 10.

### Example 8

### VSV Propagation and Infection Procedures

The Indiana strain of VSV can be obtained from the American Tissue Culture Collection, Rockville, MD, under the accession number ATCC VR-158. Due to the known pathogenicity of the virus in cattle a USDA permit is prerequisite to delivery of the culture.

The VSV culture is used to infect appropriate cell-lines for propagation and determination of plaque forming unit (PFU). BHK-21 cells (ATCC CRL 8544) were used to prepare viral stocks. Other cell-lines appropriate for this purpose include: Chick embryo fibroblasts, HeLa, BK, Vero, BHK and MK2.

VSV, ATCC VR-158 is delivered in freeze-dried form and resuspended in HBS to a titer of about 10⁵ PFU/ml. About five 75mm² polystyrene flasks comprising confluent monolayers of about 10⁷ BHK-21 cells are used to prepare the virus. Virus stocks were prepared by growing virus in BHK-21 cells at a multiplicity of infection (MOI) of ~0.1 PFU per cell and were titrated by assay of PFU on monolayers of L cells. The BHK-21 cells and L cells were grown as described in McAllister, P.E., et al., 1976, J. Virol. 18:550-558. The culture medium, Dulbecco Modified Eagle Medium (DMEM), calf serum, and horse serum were obtained from GIBCO Laboratories, Grand Island, NY. Virus was purified by sucrose and potassium tartrate gradient centrifugation as described by Barenholz et al., 1976, Biochemistry 15:3568-3570. Cell lines transformed with the illustrative plasmids of the invention as taught in Example 9 were challenged with VSV at a multiplicity of infection of ~100.

## Example 9

### Transformation of Eukaryotic Cell-Lines

#### A. Scope of Method

The transformation protocol set forth in this example utilizes the 293 cell-line to exemplify the culture conditions, DNA preparation for transformation, and the transformation procedure. This procedure was used for all experiments disclosed herein and is therefore applicable to transformations involving any of the plasmids disclosed in the present application. Transformation of the COS-1 cell-line with the plasmids of the invention proceeded in substantial accordance with the teachings of the present example.

#### B. Culture Conditions for Cell Lines

The 293 cells are obtained from the ATCC under the accession number CRL 1573 in a 25 mm$^2$ flask containing a confluent monolayer of about $5.5 \times 10^6$ cells in Eagle's Minimum Essential Medium with 10% heat-inactivated horse serum. The flask is incubated at 37°C; medium is changed twice weekly. The cells are subcultured when the cell monolayer approaches confluency by removing the medium, rinsing with Hank's Balanced Salts solution (HBS) (Gibco), adding 0.25% trypsin in HBS for 1-2 minutes, rinsing with fresh medium, aspirating, and dispensing into new flasks at a subcultivation ratio of 1:5 or 1:10.

#### C. Transformation

One day prior to transformation, cells were seeded at $0.7 \times 10^6$ cells per dish. The medium was changed 4 hours prior to transformation. Sterile, ethanol-precipitated plasmid DNA dissolved in TE buffer was used to prepare a 2X DNA-CaCl$_2$ solution containing 40 µg/ml DNA and 250 mM CaCl$_2$. 2X HBS was prepared containing 280 mM NaCl, 50 mM HEPES, and 1.5 mM sodium phosphate, with the pH adjusted to 7.05-7.15. The 2X DNA-CaCl$_2$ solution was added dropwise to an equal volume of sterile 2X HBS. A one ml sterile plastic pipette with a cotton plug was inserted into the mixing tube that contained the 2X HBS, and bubbles were introduced by blowing while the DNA was being added. The calcium-phosphate-DNA precipitate was allowed to form without agitation for 30-45 minutes at room temperature.

The precipitate was then mixed by gentle pipetting with a plastic pipette, and one ml (per plate) of precipitate was added directly to the 10 ml of growth medium that covers the recipient cells. After 4 hours of incubation at 37°C, the medium was replaced with DMEM with 10% fetal bovine serum and the cells allowed to incubate for an additional 72 hours before being challenged with VSV as described in Example 8. The cell-lines which were transformed with the plasmids of the invention and infected with VSV, were assayed for levels of protein synthesis and reporter gene expression (β-gal or CAT) to generate the data of Tables I, II and III.

## Claims

1.  A method for the selective expression of products of interest in eukaryotic cells comprising culturing an eukaryotic host cell transformed with at least one recombinant DNA expression vector comprising a viral protector sequence positioned on said recombinant DNA vector such that the protector sequence is transcribed on the mRNA encoding said product of interest and a viral suppressor sequence positioned in proper reading frame 3' to a second promoter such that the expression of the mRNA encoding said product of interest and comprising a viral protector sequence coincides with the expression of the viral suppressor.

2.  The method of Claim 1 wherein said protector sequence isolated from vesicular stomatitis virus is the leader/N gene sequence.

3.  The method of Claim 1 wherein said suppressor sequence is the N gene of vesicular stomatitis virus.

4.  A method for the isolation of viral protector sequences from DNA viruses which suppress non-viral protein synthesis consisting of:
    (a) isolating the DNA from the virus, and
    (b) preparing DNA fragments from the genomic DNA of step (a), and
    (c) constructing a recombinant DNA expression vector comprising the DNA fragments of step (b), said DNA fragments being positioned so as to result in their transcription on mRNA also encoding a readily detectable substance, and

(d) transforming an eukaryotic host cell with the recombinant DNA vector of step (c), and

(e) infecting the host cell of step (d) with the virus of step (a), and

(f) screening the viral infected host cell of step (e) to determine selective expression of the readily detectable substance, and

(g) isolating the protector sequence of the recombinant DNA vectors selectively expressing the readily detectable substance in step (f).

5. A method for isolating viral suppressor encoding sequences from double stranded DNA viruses which suppress non-viral protein synthesis consisting of:

(a) isolating the DNA from the virus, and

(b) preparing DNA fragments from the genomic DNA of step (a), and

(c) constructing recombinant DNA expression vectors comprising the DNA fragments of step (b) positioned 3′ to an eukaryotic promoter so as to result in transcription of the DNA fragment of step (b), and

(d) transforming the recombinant DNA vector of step (c) into a host cell, and

(e) assaying the host cell of step (d) for suppression of non-viral protein synthesis, and

(f) isolating the suppressor sequences from the recombinant DNA vectors of step (c) which suppress non-viral protein synthesis.

6. A method for isolating viral suppressor encoding sequences from RNA viruses consisting of:

(a) isolating the RNA from the virus, and

(b) preparing cDNA from the RNA of step (a),

(c) preparing cDNA fragments of the cDNA of step (b),

(d) constructing recombinant DNA expression vectors comprising the DNA fragments of step (c) positioned 3′ to an eukaryotic promoter as to result in transcription of the DNA fragment of step (c), and

(e) transforming the recombinant DNA vector of step (d) into a host cell, and

(f) assaying the host cell of step (e) for suppression of non-viral protein synthesis, and

(g) isolating the suppressor sequences from the recombinant DNA vectors of step (c) which suppress non-viral protein synthesis.

7. An eukaryotic promoter that consists of cDNA prepared from vesicular stomatitis virus RNA.

8. The promoter of Claim 7 that is the leader/N gene cDNA.

9. A recombinant eukaryotic DNA expression vector that comprises the VSV-derived eukaryotic promoter of Claim 8.

10. A method of producing recombinant DNA encoded polypeptide products in prokaryotic cells, said method consisting of

a) transforming a prokaryote with a recombinant DNA vector selected from the group consisting of plasmid pUCLNβ-1, pUCLNβ-2, pUCLNβ-3, pUCLNβ-5, and

b) culturing the transformed cell of step (a) under conditions appropriate for growth and expression of said polypeptide product of interest, and

c) recovering the polypeptide product of interest produced in step (b).

# FIG. I
## Restriction Site and Function Map of BK Virus

BK Virus

# FIG. 2

## Restriction Site and Function Map of Plasmid pBKE1

pBKE1

# FIG. 3
## Restriction Site and Function Map of Plasmid pBKneo1

pBKneo1

# FIG. 4

## Restriction Site and Function Map of Plasmid pSV2cat

pSV2cat

# FIG. 5
# Restriction Site and Function Map of
# Plasmid pLPcat

pLPcat

# FIG. 6
## Restriction Site and Function Map of Plasmid pBLcat

pBLcat

# FIG. 7
## RESTRICTION SITE AND FUNCTION MAP
## OF PLASMID pUCLNB-1

# FIG. 8
## RESTRICTION SITE AND FUNCTION MAP
## OF PLASMID pUCLNB-2

# FIG. 9
## RESTRICTION SITE AND FUNCTION MAP
## OF PLASMID pUCLNB-5

EcoRI      BamHI

Scal

AmpR

~ 7.3 kb

Beta-Gal

HindIII

BKV enh

AvrII
HindIII

PstI

Leader

ATG N-gene

BamHI

# FIG. 10
## RESTRICTION SITE AND FUNCTION MAP
## OF PLASMID pUCLNB-4

| European Patent Office | EUROPEAN SEARCH REPORT | Application Number |
|---|---|---|
| | | EP 91 30 8810 |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | CELL<br>vol. 36, no. 2, February 1984, CAMBRIDGE, NA US<br>pages 533 - 543;<br>GRINNELL, B.W. &WAGNER, R.R.: 'Nucleotide sequence and secondary structure of VSV leader RNA and homologous DNA involved in inhibition of DNA-dependent transcription'<br>* the whole document *<br>--- | 6 | C12N15/86<br>C12N15/47<br>C12N15/85<br>C12N15/67 |
| X | PROCEEDINGS OF THE NATIONAL ACADEMY OF SCIENCES OF USA.<br>vol. 87, June 1990, WASHINGTON US<br>pages 4499 - 4503;<br>YU, D. ET AL.: 'Transcriptional repression of the neu protooncogene by the adenovirus SEIA gene products'<br>* the whole document *<br>--- | 4 | |
| X | JOURNAL OF VIROLOGY<br>vol. 62, no. 4, April 1988,<br>pages 1286 - 1292;<br>REMENICK, J. ET AL.: 'Inhibition of the Adenovirus DNA replication by Vesicular Stomatitis Virus leader RNA'<br>* the whole document *<br>--- | 4 | TECHNICAL FIELDS SEARCHED (Int. Cl.5 )<br><br>C12N<br>C07K |
| X | CELL.<br>vol. 19, no. 2, 1980, CAMBRIDGE, NA US<br>pages 415 - 422;<br>ROSE, J.K.: 'Complete and intergenic flanking gene sequences from the genome of Stomatitis Vesicular Virus'<br>* the whole document *<br>----- | 7 | |

The present search report has been drawn up for all claims

| Place of search | Date of completion of the search | Examiner |
|---|---|---|
| THE HAGUE | 22 JANUARY 1992 | CHAMBONNET F.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document